# FASCICULE DE BREVET EUROPEEN

(11) **EP 2 198 273 B1**
(45) Date de publication et mention de la délivrance du brevet: **27.07.2022**
(21) Numéro de dépôt: 08843195.2
(22) Date de dépôt: 10.10.2008
(51) Int. Cl.: A61B 1/00, A61B 1/04, A61B 1/06, A61B 1/07, A61B 5/00, G01N 21/63, G01N 21/64, G02B 21/00, G02B 26/10, G02B 23/14

(54) **DISPOSITIF D'IMAGERIE MODULAIRE ET PROCÉDÉ D'IMAGERIE**
MODULARES BILDGEBUNGSSYSTEM UND BILDGEBUNGSVERFAHREN
MODULAR IMAGING SYSTEM AND IMAGING METHOD

(30) Priorité: 11.10.2007 FR 0758236
(43) Date de publication de la demande: 23.06.2010
(73) Titulaire: Mauna Kea Technologies, 75010 Paris (FR)
(72) Inventeur: VIELLEROBE, Bertrand, F-94130 Nogent sur Marne (FR); LACOMBE, François, F-92370 Chaville (FR); BOULAROT, Nicolas, F-94500 Champigny sur Marne (FR); DOUSSOUX, François, F-75009 Paris (FR); LAVILLONNIERE, Nicolas, F-94480 Ablon sur Seine (FR)
(74) Mandataire: IPAZ
(86) Numéro de dépôt international: PCT/FR2008/051845
(87) Numéro de publication internationale: WO 2009/053632

(56) Documents cités:
- EP-A- 1 686 407
- EP-A1- 1 811 328
- WO-A-2004/106985
- WO-A-2006/000704
- US-A- 5 659 642
- US-A1- 2006 017 920
- US-B1- 6 370 422
- KNITTEL J ET AL: "Endoscope-compatible confocal microscope using a gradient index-lens system" OPTICS COMMUNICATIONS, NORTH-HOLLAND PUBLISHING CO. AMSTERDAM, NL, vol. 188, no. 5-6, 15 février 2001 (2001-02-15), pages 267-273, XP004317426 ISSN: 0030-4018
- GMITRO A F ET AL: "CONFOCAL MICROSCOPY THROUGH A FIBER-OPTIC IMAGING BUNDLE", OPTICS LETTERS, OPTICAL SOCIETY OF AMERICA, US, vol. 18, no. 8, 15 April 1993 (1993-04-15) , pages 565-567, XP000364196, ISSN: 0146-9592

## Description

### Domaine technique

La présente invention concerne un dispositif d'imagerie par balayage proximal d'un toron de fibres optiques. Elle concerne aussi un procédé mis en œuvre par ce dispositif.

Le domaine de l'invention est notamment celui de l'endoscopie et de la microscopie confocale fibrée.

### Etat de la technique antérieure

Le document US 5 659 642 A décrit un microscope confocal comprenant un module d'illumination, un module de détection, et un module à interrupteurs relié à un guide d'image et comprenant un ensemble d'interrupteurs. Le module à interrupteurs est agencé pour commuter de façon sélective des fibres du guide d'image dans lesquelles circule un faisceau d'excitation.

Les documents US 6 370 422 B1 et « Endoscope-compatible confocal microscope using a gradient index-lens system » (Optics Communications 188 (2001) 267-273) décrivent des systèmes optiques comprenant un guide d'image qui est relié classiquement à des moyens d'illumination ou de détection par un ensemble de lentille, miroir, filtre ou cube séparateur.

Le document EP 1 686 407 A1 décrit un microscope confocal comprenant un module d'illumination, un module de détection, et un module d'injection relié à un objectif de microscope.

Le document US 2006 / 017 920 A1 décrit un microscope confocal comprenant un module d'illumination et de détection, et un module d'injection relié à un objectif de microscope.

Le document WO 2004 / 106 985 A2 décrit un système optique comprenant une fibre double.

Le document EP 1 811 328 A1 décrit un dispositif d'observation biologique comprenant un guide d'images de plusieurs fibres optiques, et une fibre optique commune à l'illumination et à la détection.

On connaît le document WO 06 000 704 A1 qui décrit un système d'imagerie microscopique de fluorescence par balayage proximal d'un toron de fibres optiques.

Un tel système comprend des lasers émettant des faisceaux d'excitation, un ensemble de filtres dichroïques, de lames séparatrices, et de lentilles qui guident les faisceaux d'excitation jusqu'à des moyens de balayage qui injectent les faisceaux d'excitation tour à tour dans une fibre d'un guide d'image et du côté proximal du guide. Le guide est agencé pour guider les faisceaux d'excitation jusqu'à son extrémité distale située en contact ou à proximité d'un échantillon. En réponse aux faisceaux d'excitation, l'échantillon émet un flux lumineux de réponse, collecté par l'extrémité distale du guide. Le flux collecté est guidé le long du guide puis par les moyens de balayage et d'injection et l'ensemble de filtres, lames, et lentilles jusqu'à un détecteur. Un trou de filtrage devant le détecteur permet de rejeter la lumière qui aurait pu être couplée dans les fibres du guide adjacentes à celle transportant les faisceaux d'excitation. Ainsi, seule la partie du flux collecté ayant été guidée le long de la fibre transportant les faisceaux d'excitation est imagée sur le détecteur.

L'alignement optique du trou de filtrage par rapport à la position des fibres du guide du côté proximal du guide est critique, car il garantit le caractère confocal du système. Un tel alignement est complexe à réaliser, car le diamètre typique des fibres du guide est de quelques micromètres. Cet alignement dépend notamment de tous les composants optiques situés entre le trou de filtrage et le guide d'image.

Les caractéristiques techniques de certains des composants optiques du système dépendent fortement des longueurs d'onde des faisceaux d'excitation utilisés, en particulier les caractéristiques des filtres dichroïques. Un premier problème de ce système est que pour changer de longueur d'onde de faisceau d'excitation, les sources laser et les filtres dichroïques sont difficilement accessibles, et il est en général préférable de remplacer le système entièrement.

Un deuxième problème d'un tel système est qu'il est quasiment impossible de remplacer ou déplacer un composant optique du système ou d'ajouter un nouveau composant optique au système sans avoir à réaligner tout le système. Notamment, sans réaligner le système, son caractère confocal risque d'être perdu.

L'objectif de la présente invention est de proposer un dispositif permettant de résoudre tout ou partie des problèmes discutés ci-dessus, ainsi qu'un procédé mis en œuvre dans un tel dispositif.

### Exposé de l'invention

Cet objectif est atteint avec un dispositif d'imagerie selon la revendication 1.

Dans ce document, deux objets sont dits reliés ou conjugués optiquement lorsqu'au moins un flux, signal ou faisceau optique peut être guidé d'un objet à l'autre. Un de ces objets peut par exemple consister en un des modules ou en une des fibres du dispositif selon l'invention.

Dans ce document, quand deux objets sont conjugués optiquement par un élément optique principal (cet élément principal comprenant typiquement une fibre optique), il peut y avoir d'autres éléments optiques intermédiaires conjuguant les objets à l'élément optique principal. La fibre de conjugaison peut comprendre une fibre d'illumination, une fibre de détection, ou une fibre de séparation qui seront décrites par la suite.

Le dispositif selon l'invention peut comprendre des moyens pour déconnecter et reconnecter la conjugaison par la fibre optique de conjugaison, comme un connecteur.

Les moyens d'émission peuvent comprendre plusieurs sources émettant chacune un faisceau d'excitation, et le module d'illumination peut comprendre des moyens pour multiplexer les faisceaux d'excitation dans la fibre optique d'illumination. Chaque source peut être conjuguée optiquement aux moyens de multiplexage par une fibre optique source, et les moyens de multiplexage peuvent comprendre par exemple un multiplexeur fibré fusionnant les cœurs des fibres source, ou des moyens de multiplexage non fibrés comprenant par exemple des systèmes acoustooptiques de multiplexage, ou encore un multiplexeur du type phasar. De préférence, la fibre optique d'illumination est agencée pour être conjuguée optiquement tour à tour à une unique fibre du guide dans laquelle est injecté tour à tour l'au moins un faisceau d'excitation par les moyens de balayage et d'injection.

De manière générale, le module de détection peut comprendre des moyens pour démultiplexer en longueur d'onde le flux lumineux collecté. Les moyens de détection peuvent comprendre plusieurs détecteurs, chaque détecteur étant agencé pour détecter une bande de longueur d'onde donnée du flux démultiplexé.

Les moyens de séparation peuvent comprendre un filtre dichroïque, de préférence multibande. Les moyens de séparation peuvent aussi comprendre un cube séparateur tel de préférence un cube polarisant, le module d'illumination et le module de détection étant alors de préférence prévus notamment pour de l'imagerie par réflectance d'un échantillon.

Suivant encore un autre aspect de l'invention, il est proposé un procédé d'imagerie selon la revendication 13.

Dans ce document, une étape de liaison ou conjugaison optique entre deux objets comprend un guidage d'au moins un flux, signal ou faisceau optique d'un objet à l'autre. Un de ces objets peut par exemple consister en un des modules ou en une des fibres du dispositif selon l'invention.

Le procédé selon l'invention peut aussi comprendre une superposition spatiale ou un multiplexage de plusieurs faisceaux d'excitation dans la fibre d'illumination.

Le procédé selon l'invention peut comprendre en outre un démultiplexage en longueur d'onde, par le module de détection, du flux lumineux collecté.

Par couplage entre les fibres d'un guide d'image, on entend une transmission, le long du guide, de lumière entre une première fibre du guide et une deuxième fibre du guide voisine de la première fibre. De manière générale, on omet dans la description de l'invention d'éventuels effets de couplage entre les fibres du guide d'image, notamment lorsqu'il est dit que la fibre optique de conjugaison est conjuguée optiquement à une unique fibre du guide : on ne considère pas le couplage entre cette unique fibre et des fibres du guide voisines de cette unique fibre. De manière préférentielle, le guide d'image du dispositif, procédé, ou module selon l'invention est agencé pour qu'il n'y ait sensiblement pas de couplage entre les fibres de ce guide.

### Description des figures et modes de réalisation

D'autres particularités et avantages de l'invention apparaîtront à la lecture de la description détaillée de modes de réalisation et de mise en œuvre nullement limitatifs, et des dessins annexés suivants, où :
- la figure 1 est une vue schématique d'un dispositif non-revendiqué,
- la figure 2 est une vue plus détaillée d'un premier mode de réalisation de dispositif non-revendiqué,
- la figure 3 est une vue d'un deuxième mode de réalisation de dispositif non-revendiqué,
- la figure 4 est une vue d'un troisième mode de réalisation de dispositif non-revendiqué,
- la figure 5 est une vue d'un quatrième mode de réalisation de dispositif selon l'invention, et
- la figure 6 est une vue d'un cinquième mode de réalisation de dispositif selon l'invention.

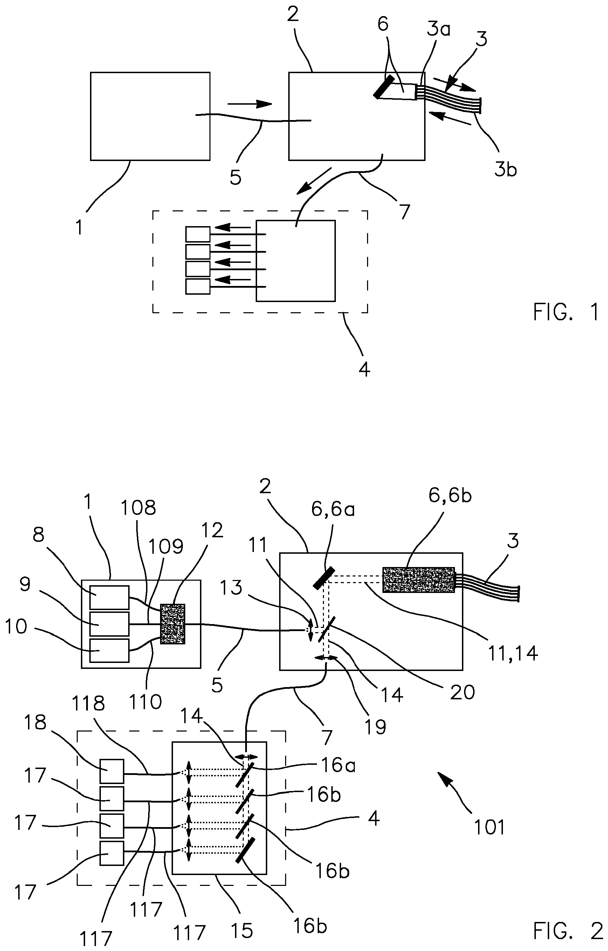

On va tout d'abord décrire, en référence à la figure 1, un dispositif dont les caractéristiques sont communes aux différents modes de réalisation qui seront décrits par la suite et qui mettent en œuvre un procédé selon l'invention.

Le dispositif comprend un module d'illumination 1, un module de balayage et d'injection 2, et un module de détection 4. Typiquement, le guide comprend plusieurs milliers de fibres d'un diamètre de quelques micromètres chacune.

Le module d'illumination 1 comprend des moyens pour émettre au moins un faisceau d'excitation, et est conjugué optiquement au module de balayage et d'injection 2 par l'intermédiaire d'une fibre optique d'illumination 5. Lorsque le module d'illumination 1 émet l'au moins un faisceau d'excitation, ce faisceau d'excitation est guidé le long de la fibre d'illumination 5 jusqu'au module de balayage et d'injection 2.

Le module de balayage et d'injection comprend un guide d'image 3 comprenant deux extrémités respectivement proximale 3a et distale 3b reliées par une pluralité de fibres optiques multimodes. Le module de balayage et d'injection 2 comprend en outre des moyens 6 pour balayer et injecter l'au moins un faisceau d'excitation tour à tour dans une fibre du guide d'image 3 et du côté proximal 3a du guide. On entend dans ce document par « position des moyens de balayage et d'injection » un état des moyens 6 de balayage et d'injection pour lequel ces moyens sont agencés pour injecter l'au moins un faisceau d'excitation dans une fibre donnée du guide 3, les moyens de balayage et d'injection prenant successivement plusieurs positions de manière à balayer périodiquement toutes les fibres du guide. Cet au moins un faisceau d'excitation est alors guidé dans cette fibre du guide jusqu'à l'extrémité distale 3b du guide 3. L'extrémité distale 3b est prévue pour être placée dans ou en contact avec un échantillon, et pour collecter un flux lumineux émis par l'échantillon. Le flux collecté peut par exemple comprendre des signaux de réflectance et/ou de fluorescence, émis par l'échantillon respectivement par réflectance et/ou par fluorescence en réponse à l'au moins un faisceau d'excitation que cet échantillon a reçu. On entend par réflectance une émission de lumière par diffusion ou par rétro diffusion. Le guide 3 forme ainsi typiquement une sonde endoscopique, l'extrémité distale 3b pouvant ou non être équipée d'une tête optique.

Le module de balayage et d'injection 2 est conjugué optiquement au module de détection 4 par l'intermédiaire d'une fibre optique de détection 7. Le flux lumineux collecté par l'extrémité distale 3b est guidé jusqu'à l'extrémité proximale 3a, puis est guidé vers l'entrée de la fibre optique de détection 7. Le flux lumineux collecté est alors guidé le long de la fibre de détection 7 jusqu'au module de détection 4.

Le module de détection 4 comprend des moyens pour détecter le flux lumineux collecté à l'extrémité distale 3b.

Une première fonction des fibres d'illumination 5 et de détection 7 conjuguant les modules 1, 2, 4 est de jouer un rôle de pont entre ces modules, et donc de séparer les fonctions internes principales du dispositif, c'est-à-dire les fonctions d'illumination, de balayage et d'injection, et de détection, en associant chacune de ces fonctions internes à un des modules 1, 2, 4. Ainsi, ces fonctions séparées sont plus simples à réparer. L'utilisation des fibres 5, 7 permet un accès simple aux composants du dispositif, par exemple pour remplacer un composant optique à l'intérieur d'un des modules 1, 2, 4 ou pour aligner entre eux des composants optiques à l'intérieur d'un des modules sans pour autant risquer de désaligner optiquement tout le dispositif.

De plus, les extrémités des fibres 5, 7 sont fixées de manière amovible à des connecteurs, de sorte que la conjugaison entre le module d'illumination 1 et le module de balayage et d'injection 2 par l'intermédiaire de la fibre d'illumination 5, et la conjugaison entre le module de détection 4 et le module de balayage et d'injection 2 par l'intermédiaire de la fibre de détection 7 peuvent être déconnectées et reconnectées. Les différents modules 1, 2, 4 du dispositif selon l'invention peuvent donc être séparés du reste du dispositif. Ainsi, les fonctions séparées sont plus simples à remplacer. Un des modules 1, 2, 4 peut donc être remplacé rapidement, sans avoir à remplacer tout le dispositif ce qui représente un avantage de coût, et sans avoir à réaligner optiquement les modules entre eux.

Les conjugaisons fibrées 5, 7 entre modules 1, 2, 4, permettent en outre d'ajouter d'autres modules au dispositif selon l'invention, ou plus généralement de faire évoluer le dispositif sans avoir à le remplacer dans son intégralité. On peut par exemple remplacer la fibre d'illumination 5 par un ensemble de composants optiques conjugué optiquement au module d'illumination 1 par une fibre optique et conjugué optiquement au module de balayage et d'injection 2 par une autre fibre optique, ou remplacer la fibre de détection 7 par un ensemble de composants optiques conjugué optiquement au module de détection 4 par une fibre optique et conjugué optiquement au module de balayage et d'injection 2 par une autre fibre optique.

Ainsi, l'architecture sous la forme de modules associés à des fonctions séparées et conjugués optiquement par des fibres optiques permet de réduire les coûts et les délais de fabrication du dispositif selon l'invention et d'améliorer la maintenance, la réparation ou l'upgrade du dispositif selon l'invention.

De plus, la fibre optique d'illumination 5 est une fibre optique monomode de préférence gaussien, le mode de la fibre monomode 5 étant sélectionné pour exciter efficacement un ou plusieurs modes du guide 3. Ainsi, la fibre 5 assure à l'au moins un faisceau d'excitation une grande qualité monomode gaussien. De plus, le taux d'injection de l'au moins un faisceau d'excitation dans le guide d'image 3 est optimal, car la PSF (« Point Spread Function » ou « fonction d'étalement ponctuel ») à l'injection dans le guide est prévu pour correspondre bien au mode fondamental des fibres optiques du guide. Ainsi, une autre fonction de la fibre d'illumination est un filtrage modal de l'au moins un faisceau d'excitation.

Enfin, la fibre optique d'illumination 5 est agencée, pour une position donnée des moyens de balayage et d'injection, pour être conjuguée optiquement à une unique fibre du guide 3, plus particulièrement à la fibre du guide dans laquelle les moyens de balayage et d'injection sont agencés pour injecter l'au moins un faisceau d'excitation. Pour une position donnée des moyens de balayage et d'injection 6, l'au moins un faisceau d'excitation transporté par la fibre illumination 5 n'est injecté que dans une unique fibre du guide. En particulier, le diamètre de la fibre d'illumination 5 et la position de l'extrémité de la fibre d'illumination 5 orientée vers le module de balayage et d'injection 2 dépendent du diamètre des fibres du guide 3, de la position de l'extrémité proximale 3a du guide et des caractéristiques des composants optiques situés entre la fibre d'illumination 5 et les moyens de balayage et d'injection 6.

La fibre optique de détection 7 est une fibre optique multimode. En effet, le flux lumineux collecté par l'extrémité distale 3b, émis par exemple par réflectance ou par fluorescence, excite en général de nombreux modes des fibres du guide. Ainsi, la fibre de détection 7 transporte le flux lumineux collecté par le guide 3 quasiment sans perte de signal. Ainsi, une autre fonction de la fibre de détection 7 est de ne pas effectuer de filtrage modal sur le flux collecté par le guide 3.

De plus, la fibre optique de détection 7 est agencée, pour une position donnée des moyens de balayage et d'injection, pour être conjuguée optiquement à une unique fibre du guide 3. Autrement dit, pour une position donnée des moyens de balayage et d'injection 6, la fibre de détection 7 transporte vers le module de détection uniquement la partie du flux lumineux collecté à l'extrémité distale 3b ayant été guidée le long de cette unique fibre du guide 3. La fibre de détection 7 permet de rejeter les parties du flux collecté ayant été guidées le long des autres fibres du guide. Comme pour la fibre d'illumination 5, le diamètre et la position de l'extrémité de la fibre de détection 7 orientée vers le module de balayage et d'injection doivent pour cela être ajustés. De manière préférentielle, la fibre optique de détection est, pour une position donnée des moyens de balayage et d'injection, conjuguée optiquement à la fibre du guide dans laquelle les moyens de balayage et d'injection injectent l'au moins un faisceau d'excitation. La fibre de détection 7 permet donc, pour une position donnée des moyens de balayage et d'injection, de rejeter les parties du flux collecté ayant été guidées le long des fibres autres que celle guidant les faisceaux d'excitation. Ainsi, la fibre de détection 7 fait office de trou de filtrage. La fibre de détection 7 a donc une fonction de filtrage spatial du flux collecté à l'extrémité distale 3b. La fibre de détection 7 permet de sélectionner la fibre du guide 3 qui a servi à l'illumination (c'est-à-dire au transport de l'au moins un faisceau d'excitation) permettant ainsi au dispositif selon l'invention de conserver son caractère confocal. Ce caractère confocal est initialement du à une conjugaison optique d'un point de l'échantillon avec uniquement la fibre du guide guidant un faisceau d'excitation excitant ce point de l'échantillon.

Les moyens de balayage et d'injection 6 sont agencés pour injecter l'au moins un faisceau d'excitation tour à tour dans une fibre du guide d'image 3, de manière à balayer périodiquement toutes les fibres du guide. En injectant tour à tour les faisceaux d'excitation dans une fibre du guide 3, les moyens de détection du module de détection 4 détectent tour à tour le flux lumineux collecté par l'extrémité distale 3b, guidé le long de cette fibre du guide, et provenant d'un point source de l'échantillon. Quand les moyens de balayage et d'injection ont balayé toutes les fibres du guide, les moyens de détection ont balayé un ensemble de points sources de l'échantillon, c'est-à-dire tout un champ de vision de l'échantillon. Les moyens de détection sont reliés à des moyens pour construire, à partir de ce balayage du champ de vision, une image de l'échantillon. Les moyens de construction peuvent construire une image de réflectance ou de fluorescence de l'échantillon, selon la nature des signaux du flux lumineux collecté. Les moyens de détection sont en outre reliés à des moyens pour visualiser l'image construite. La taille du champ de vision dépend du nombre de fibres dans le guide d'image, du diamètre de ces fibres, et éventuellement des caractéristiques de la tête optique située à l'extrémité distale du guide si cette tête existe. Typiquement, le guide comprend quelques milliers de fibres ayant un diamètre de quelques micromètres. Le champ de vision fait donc typiquement une ou quelques centaines de micromètres de côté. Le dispositif selon l'invention est alors particulièrement adapté pour de la microscopie confocale fibrée de fluorescence et/ou de réflectance.

On va maintenant décrire, en référence à la figure 2, un premier mode de réalisation de dispositif 101 appliqué notamment à de la détection, quantification ou de l'imagerie de fluorescence. Ce mode de réalisation ayant toutes les caractéristiques du dispositif venant d'être décrit en référence à la figure 1, les références 1 à 7 ne seront pas de nouveau décrites.

Les moyens d'émission du module d'illumination 1 comprennent plusieurs sources d'excitation 8, 9, 10. Ces sources d'excitation sont des sources laser qui émettent chacune un faisceau d'excitation ayant une longueur d'onde ou bande de longueur d'onde différente de celles des autres faisceaux d'excitation. Le dispositif 101 comprend en outre des moyens pour multiplexer les sources 8, 9, 10 au sein de la fibre optique d'illumination 5. Ainsi, la fibre optique d'illumination 5 permet de superposer spatialement tous les faisceaux d'excitation 11 en entrée du module de balayage et d'injection 2.

Chaque source 8, 9, 10 est conjuguée optiquement à un multiplexeur 12 par une fibre source respectivement 108, 109 ou 110 qui transporte le faisceau d'excitation émis par ladite source. Le multiplexeur 12 est un multiplexeur fibré agencé pour fusionner les coeurs des fibres sources 108 à 110 en une unique fibre dont la fibre d'illumination 5 est le prolongement. En entrée du module de balayage et d'injection 2, les faisceaux d'excitation 11 sont collimatés par un système optique 13.

Pour les mêmes raisons que pour la fibre d'illumination 5, les fibres sources 108 à 110 sont des fibres monomodes. Ainsi, les fibres 108 à 110 assurent à chaque faisceau d'excitation une grande qualité monomode gaussien, et un taux optimal d'injection de chaque faisceau d'excitation dans le guide d'image 3. Chaque fibre source permet donc un filtrage modal d'un faisceau d'excitation.

Le module d'illumination 1 comprend un boîtier à l'intérieur duquel sont regroupées les sources 8 à 10, les fibres sources 108 à 110 et le multiplexeur 12. La fibre d'illumination 5 peut être déconnectée et reconnectée du boîtier du module d'illumination 1 au moyen d'un connecteur. De même, la fibre source 108 à 110 peut être déconnectée et reconnectée de la source respectivement 8 à 10, ce qui permet de remplacer cette source et donc la longueur d'onde ou bande de longueur d'onde du faisceau d'excitation émis par cette source.

Le module de détection 4 comprend des moyens pour démultiplexer en longueur d'onde le flux lumineux 14 collecté à l'extrémité distale du guide 3. Les moyens de démultiplexage 15 comprennent plusieurs filtres dichroïques 16a, 16b. Chaque filtre dichroïque 16a ou 16b renvoi vers un détecteur 17 ou 18 une bande différente de longueur d'onde du flux 14. Ainsi, chaque détecteur 17 ou 18 est agencé pour détecter une bande différente de longueur d'onde du flux 14, et permet d'imager l'échantillon à l'aide de signaux de fluorescence dont la longueur d'onde est comprise dans cette bande et collectés par l'extrémité distale 3b.

Les moyens de détection comprennent au moins un détecteur 17 ou 18 par source 8 à 10 et donc par bande de longueur d'onde de faisceau d'excitation. Chaque détecteur 17 ou 18 est associé à un faisceau d'excitation. En effet, la bande de longueur d'onde de chaque détecteur 17 ou 18 correspond de préférence à la bande d'émission d'un fluorophore excité à la longueur d'onde ou bande de longueur d'onde du faisceau d'excitation associé audit détecteur. Sur l'exemple illustré à la figure 2, le dispositif 101 comprend trois sources 8 à 10 et quatre détecteurs 17 et 18, car plusieurs bandes de longueurs d'onde du flux 14 peuvent être liées à une même longueur d'onde ou bande de longueur d'onde de faisceau d'excitation, ledit faisceau d'excitation pouvant être adapté pour exciter plusieurs fluorophores.

Chaque filtre dichroïque 16a ou 16b est associé à une fibre de démultiplexage 117 ou 118 agencée pour guider vers le détecteur 17 ou 18 la bande de longueurs d'onde renvoyée par ce filtre dichroïque. Pour les mêmes raisons que la fibre de détection 7, les fibres de démultiplexage sont des fibres optiques multimodes. Pour des raisons de photométrie, et pour un grandissement optique égal à un de l'ensemble de composants optiques reliant optiquement chaque fibre de démultiplexage avec la fibre de détection 7, les fibres de démultiplexage ont chacune un diamètre supérieur ou égal (de préférence supérieur) au diamètre de la fibre de détection 7, ce qui permet une plus grande tolérance d'alignement optique, et permet en outre de collecter un maximum de flux c'est-à-dire de limiter les pertes photométriques. Si le grandissement optique est différent de un, les fibres de démultiplexage ont chacune un diamètre supérieur ou égal (de préférence supérieur) au diamètre de la fibre de détection 7 ajusté par ce grandissement optique.

La fibre de détection 7 peut être connectée et déconnectée du module de détection 4 au moyen d'un connecteur. De même, le module de détection comprend des moyens pour déconnecter et reconnecter chaque fibre de multiplexage 117, 118, ce qui permet de remplacer simplement les détecteurs 17 et 18.

Le module de balayage et d'injection 2 comprend un boîtier à l'intérieur duquel sont regroupés différents systèmes optiques 13, 19, les moyens de balayage et d'injection 6, et des moyens pour séparer le chemin optique des faisceaux d'excitation 11 du chemin optique du flux collecté 14. Le guide d'image 3 est déconnectable du boîtier du module de balayage et d'injection 2, ce qui permet notamment de changer le type du guide 3 connecté au boîtier du module de balayage et d'injection 2. Comme différents types de guide 3, on peut citer par un exemple un guide dont les fibres sont multimodes, un guide avec une tête optique à son extrémité distale, un guide sans tête optique à son extrémité distale, un guide avec un nombre de fibres donné, un guide avec un diamètre de fibres donné, ou encore un guide avec une longueur donnée entre son extrémité proximale et son extrémité distale.

Les moyens de séparation comprennent un filtre dichroïque 20. Le filtre 20 est un filtre multibande, c'est-à-dire qu'il peut réfléchir plusieurs bandes de longueur d'onde différentes et peut transmettre plusieurs autres bandes de longueurs d'onde. Sur l'exemple illustré à la figure 2, le filtre 20 réfléchit des bandes correspondant aux longueurs d'onde des faisceaux d'excitation 11, et transmet des bandes correspondant à des longueurs d'onde du flux collecté 14 et aux bandes réfléchies par les filtres 16a, 16b et détectées par les détecteurs 17 et 18. On peut aussi réaliser le dispositif 101 avec un filtre 20 qui réfléchit des bandes correspondant à des longueurs d'onde du flux collecté 14 et aux bandes réfléchies par les filtres 16a, 16b et détectées par les détecteurs 17 et 18, et transmet des bandes correspondant aux longueurs d'onde des faisceaux d'excitation, à condition d'échanger les positions de la fibre d'illumination 5 et du module d'illumination 1 avec les positions de la fibre de détection 7 et du module de détection 4 et d'intervertir les systèmes optiques 13 et 19.

Les faisceaux lumineux d'excitation 11 guidés le long de la fibre d'illumination 5 et collimatés par le système optique 13 sont dirigés par les moyens de séparation vers les moyens de balayage et d'injection 6. Les moyens de balayage et d'injection 6 comprennent deux miroirs mobiles 6a permettant un balayage bidimensionnel dans le plan de la surface d'entrée de l'extrémité proximale du guide, et un système optique 6b. Les miroirs mobiles 6a injectent les faisceaux d'excitation tour à tour dans une fibre du guide d'image. Avant d'entrer dans le guide 3, les faisceaux d'excitation passent à travers le système optique 6b qui les focalise sur l'extrémité proximale du guide 3.

À l'inverse, le flux collecté 14 est collimaté par le système optique 6b puis est dirigé par les miroirs mobiles 6a vers le module de détection 4. Entre les miroirs mobiles 6a et le module de détection 4, le flux collecté 14 passe à travers les moyens de séparation, est focalisé par le système optique 19 en entrée de la fibre de détection 7, puis est guidé le long de la fibre de détection 7 jusqu'au module de détection 4.

La fibre d'illumination 5 peut être déconnectée et reconnectée du boîtier du module de balayage et d'injection 2 au moyen d'un connecteur. De même, la fibre de détection 7 peut être déconnectée et reconnectée du boîtier du module de balayage et d'injection 2 au moyen d'un connecteur.

Un problème du dispositif 101 est la caractéristique spectrale du filtre dichroïque 20 situé à l'intérieur du boîtier du module de balayage et d'injection 2, notamment les bandes de longueur d'onde qu'il doit transmettre ou réfléchir, dépendent des longueurs d'onde des faisceaux d'excitation et du flux collecté. Un changement de longueur d'onde d'un des faisceaux d'excitation impose de changer le filtre multibande 20, alors que celui-ci est compliqué à spécifier, à fabriquer et à aligner optiquement dans le module de balayage et d'injection.

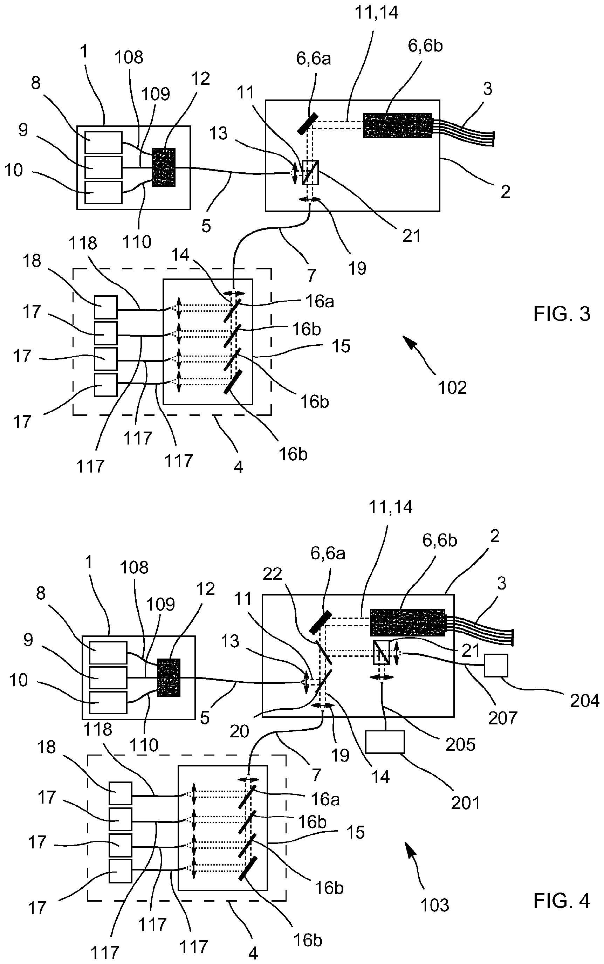

On va maintenant décrire, en référence à la figure 3, un deuxième mode de réalisation de dispositif 102 qui permet de résoudre ce problème. Le dispositif 102 ne sera décrit que pour ses différences par rapport au dispositif 101 décrit en référence à la figure 2. En particulier, les références 1 à 19, 108 à 110, 117 et 118 ne seront pas de nouveau décrites. Le dispositif 102 permet notamment la détection de signaux de fluorescence ou de réflectance collectés à l'extrémité distale du guide, et permet de quantifier ces signaux et/ou de construire des images de l'échantillon par fluorescence et par réflectance à partir de ces signaux.

Dans le dispositif 102, les moyens de séparation du module de balayage et d'injection 2 consistent en un cube polarisant 21 qui remplace le filtre dichroïque multibande. Le cube polarisant 21 réfléchit des faisceaux ayant une première polarisation, et transmet les faisceaux ayant une polarisation orthogonale à la première polarisation. Tous les faisceaux d'excitation 11 ont une même polarisation. Les réflexions parasites des faisceaux d'excitation, par exemple au niveau des moyens de balayage et d'injection 6 ou du guide 3, ont conservé la même polarisation que celle des faisceaux d'excitation. Par contre, le flux collecté 14 provenant de la partie distale du guide 3 a perdu l'état de polarisation des faisceaux d'excitation, et comprend donc des signaux ayant une ou plusieurs polarisations aléatoires dans le temps. Le cube polarisant 21 est agencé pour diriger les faisceaux d'excitation 11 vers les moyens de balayage et d'injection 6, et pour diriger le flux lumineux collecté 14 vers le module de détection 4. Sur l'exemple de la figure 3, le cube 21 réfléchit les faisceaux d'excitation 11 ainsi que les réflexions parasites, et transmet du moins en partie le flux collecté 14. On peut aussi réaliser le dispositif 102 avec un cube 21 qui réfléchit au moins en partie le flux collecté 14, et transmet les faisceaux d'excitation, à condition d'échanger les positions de la fibre d'illumination 5 et du module d'illumination 1 avec les positions de la fibre de détection 7 et du module de détection 4. Ainsi, le cube 21 permet de faire une séparation entre d'une part les faisceaux d'excitation et les réflexions parasites ayant conservé la polarisation des faisceaux d'excitation, et d'autre part le flux collecté 14 provenant de la partie distale du guide 3.

Un des filtres 16a du module de détection 4 est réalisé pour diriger vers le détecteur 18 une bande de longueurs d'onde du flux collecté 14 comprenant la longueur d'onde ou bande de longueur d'onde d'une des sources laser 8. Ainsi, le détecteur 18 et la source laser 8 peuvent être utilisés pour de l'imagerie de réflectance de l'échantillon situé au niveau de l'extrémité distale du guide. Cela est uniquement possible du fait qu'il y a une réjection des réflexions parasites par le cube 21. Les autres filtres 16b du module de détection 4 et les détecteurs 17 associés permettent, comme pour le premier mode de réalisation, de quantifier des signaux de fluorescence en provenance de l'échantillon ou de faire de l'imagerie de fluorescence de l'échantillon.

L'utilisation du cube 21 présente un désavantage par rapport au premier mode de réalisation de dispositif. En effet, l'utilisation du cube 21 entraîne des pertes d'intensité du flux collecté 14, et en particulier des pertes d'intensité de signaux de fluorescence. En effet, si on considère que le cube dirige toute l'intensité des faisceaux d'excitation 11 vers les moyens de balayage et d'injection 6 du fait de leur polarisation, le flux collecté 14 est en partie réfléchie et en partie transmis par le cube 21 du fait qu'il ne possède pas de polarisation unique. Le flux collecté 14 n'est donc dirigé qu'en partie vers le module de détection 4. Typiquement, on perd 50 % de l'intensité du flux collecté 14 et dirigé vers le module de détection 4. Ainsi, on diminue la sensibilité du dispositif 102.

De plus, pour ne pas perdre d'intensité des faisceaux d'excitation 11 dirigés vers les moyens de balayage et d'injection, toutes les fibres guidant les faisceaux d'excitation 11 jusqu'aux moyens de balayage et d'injection 6 (c'est-à-dire les fibres sources 108 à 110 et la fibre d'illumination 5) sont des fibres à maintien de polarisation, ce qui entraîne un surcoût de réalisation et un réglage spécifique.

On va maintenant décrire, en référence à la figure 4, un troisième mode de réalisation de dispositif 103. Le dispositif 103 ne sera décrit que pour ses différences par rapport au dispositif 101 décrit en référence à la figure 2. En particulier, les références 1 à 20, 108 à 110, 117 et 118 ne seront pas de nouveau décrites. Le dispositif 102 permet notamment de la détection de signaux de fluorescence et/ou de réflectance provenant collecté à l'extrémité distale du guide, et permet de quantifier ces signaux et de construire, à partir de ces signaux, des images de l'échantillon par fluorescence et par réflectance.

Le dispositif 103 comprend en outre un deuxième module d'illumination 201 et un deuxième module de détection 204. Le deuxième module d'illumination 201 diffère du module d'illumination 1 qu'en ce qu'il ne comprend qu'une unique source émettant un faisceau d'excitation, et ne comprend pas de moyens de multiplexage. Le deuxième module de détection 204 diffère du module de détection 4 qu'en ce qu'il ne comprend qu'un seul détecteur, pas de filtres et pas de moyens de démultiplexage.

Les deuxièmes module d'illumination 201 et de détection 204 sont conjugués optiquement aux moyens de balayage et d'injection 6 de la même manière que les modules d'illumination et de détection du deuxième mode de réalisation 102 illustré sur la figure 3. En effet, le deuxième module d'illumination 201 est conjugué optiquement au module de balayage et d'injection 2 par l'intermédiaire d'une deuxième fibre optique d'illumination monomode 205 à maintien de polarisation, le deuxième module de détection de 204 est conjugué optiquement au module de balayage et d'injection 2 par l'intermédiaire d'une deuxième fibre optique de détection multimode 207, et le module de balayage et d'injection 2 comprend le cube polarisant 21 déjà décrit en référence à la figure 3. Le cube 21 est agencé pour diriger d'une part le faisceau d'excitation du deuxième module d'illumination 201 vers les moyens de balayage et d'injection 6 et pour diriger le flux lumineux collecté 14 en partie vers le deuxième module de détection 204.

Le filtre dichroïque 20 et le cube polarisant 21 sont conjugués optiquement aux moyens de balayage et d'injection 6 par une même lame séparatrice 22. La lame séparatrice 22 est agencée pour diriger vers les moyens de balayage et d'injection 6 les faisceaux d'excitation provenant du premier ou du deuxième module d'illumination. La lame séparatrice 22 est en outre agencée pour diriger le flux collecté 14 en partie vers le premier module de détection 4 et en partie vers le deuxième module de détection 204. La partie du flux 14 dirigée vers le premier module de détection 4 comprend les bandes de longueur d'onde du flux 14 dirigées par les filtres 16a, 16b vers les détecteurs 17, 18. La partie du flux 14 dirigée vers le deuxième module de détection 204 comprend la longueur d'onde ou bande de longueur d'onde du faisceau d'excitation émis par le deuxième module d'illumination 201, de sorte que le détecteur du deuxième module de détection 204 est agencé pour détecter au sein du flux collecté 14 un signal de réflectance émis par l'échantillon en réponse au faisceau d'excitation du deuxième module d'illumination 201. Ainsi, le deuxième module de détection 204 permet de réaliser de l'imagerie de réflectance de l'échantillon. En effet, des réflexions parasites du faisceau d'excitation du deuxième module d'illumination 201 s'opèrent notamment dans les moyens de balayage et d'injection, mais la réjection de ces réflexions parasitent est assurée par le cube polarisant 21. Le dispositif 103 permet donc de réaliser, par microscopie confocale fibrée, simultanément de l'imagerie de réflectance (et donc de l'imagerie morphologique), grâce au deuxième module d'illumination et au deuxième module de détection, et de l'imagerie de fluorescence (et donc de l'imagerie fonctionnelle), grâce au premier module d'illumination et au premier module de détection, de l'échantillon situé à proximité ou en contact de l'extrémité distale du guide 3.

Dans une première variante les moyens de balayage et d'injection 6 sont agencés, au cours de ses différentes positions successives, pour conjuguer optiquement la première fibre optique d'illumination 5, la première fibre optique de détection 7, la deuxième fibre optique d'illumination 205, et la deuxième fibre optique de détection 207 tour à tour avec une unique fibre du guide d'image 3, de manière à balayer périodiquement toutes les fibres du guide:
- la première fibre optique d'illumination 5 est agencée, pour une position donnée des moyens de balayage et d'injection (c'est-à-dire à un instant donné), pour être conjuguée optiquement à une première unique fibre du guide 3.
- la première fibre optique de détection 7 est agencée, pour cette même position donnée des moyens de balayage et d'injection, pour être conjuguée optiquement à cette première unique fibre du guide 3,
- la deuxième fibre optique d'illumination 205 est agencée, pour cette même position donnée des moyens de balayage et d'injection, pour être conjuguée optiquement à cette première unique fibre du guide 3, et
- la deuxième fibre optique de détection 207 est agencée, pour cette même position donnée des moyens de balayage et d'injection, pour être conjuguée optiquement à cette première unique fibre du guide 3.

Cette première variante permet de simplifier la construction des images de réflectance et de fluorescence.

Dans une deuxième variante les moyens de balayage et d'injection 6 sont agencés, au cours de ses différentes positions successives, pour conjuguer optiquement la première fibre optique d'illumination 5 et la première fibre optique de détection 7 tour à tour avec une unique fibre du guide d'image 3 de manière à balayer périodiquement toutes les fibres du guide, et pour conjuguer optiquement la deuxième fibre optique d'illumination 205 et la deuxième fibre optique de détection 207 tour à tour avec une autre unique fibre du guide d'image 3 de manière à balayer périodiquement toutes les fibres du guide:
- la première fibre optique d'illumination 5 est agencée, pour une position donnée des moyens de balayage et d'injection (c'est-à-dire à un instant donné), pour être conjuguée optiquement à une première unique fibre du guide 3.
- la première fibre optique de détection 7 est agencée, pour cette même position donnée des moyens de balayage et d'injection, pour être conjuguée optiquement à cette première unique fibre du guide 3,
- la deuxième fibre optique d'illumination 205 est agencée, pour cette même position donnée des moyens de balayage et d'injection, pour être conjuguée optiquement à une deuxième unique fibre du guide 3, de préférence voisine de la première unique fibre, et
- la deuxième fibre optique de détection 207 est agencée, pour cette même position donnée des moyens de balayage et d'injection, pour être conjuguée optiquement à cette deuxième unique fibre du guide 3.

Cette deuxième variante permet de réduire encore plus les réflexions parasites entre les signaux de réflectance et les signaux de fluorescence, mais nécessite un recalage temporel des images de réflectance et de fluorescence construites.

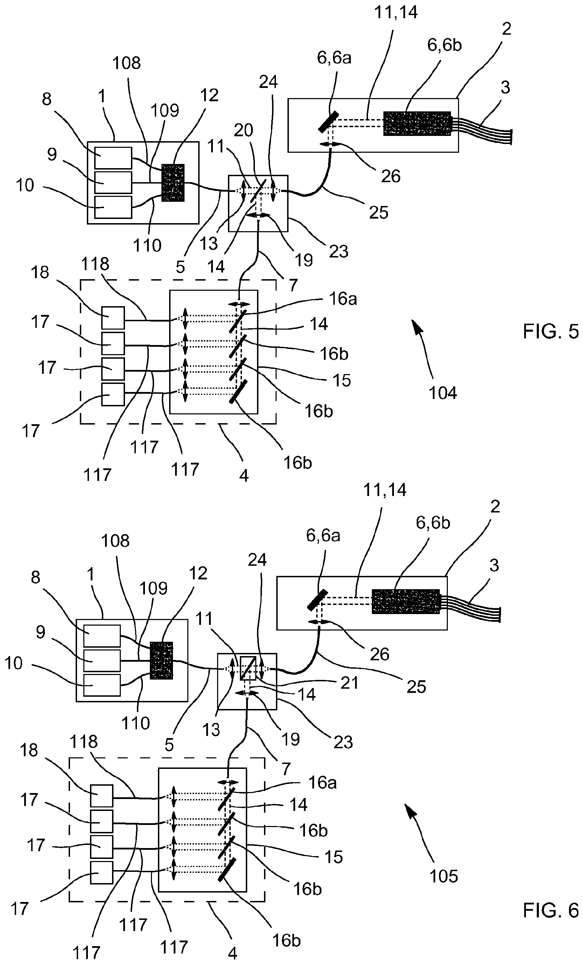

On va maintenant décrire, en référence à la figure 5, un quatrième mode de réalisation de dispositif 104 selon l'invention, appliqué notamment à de la détection, quantification ou de l'imagerie de fluorescence de l'échantillon. Le dispositif 104 ne sera décrit que pour ses différences par rapport au dispositif 101 décrit en référence à la figure 2. En particulier, les références 1 à 20, 108 à 110, 117 et 118 ne seront pas de nouveau décrites.

Contrairement au premier mode de réalisation 101 décrit en référence à la figure 2, les moyens 20 pour séparer le chemin optique des faisceaux d'excitation 11 du chemin optique du flux collecté 14, et les systèmes optiques 13 et 19 sont situés en dehors du module de balayage et d'injection 2. Ainsi, le module de balayage et d'injection est totalement indépendant des longueurs d'onde des faisceaux d'excitation 11 et du flux collecté 14.

En effet, le dispositif 104 comprend un module de séparation 23. Le module de séparation 23 comprend un boîtier à l'intérieur duquel sont regroupés les systèmes optiques 13, 19, un autre système optique 24, et le filtre dichroïque 20.

La fibre d'illumination 5 peut être déconnectée et reconnectée non plus au boitier du module de balayage et d'injection, mais au boîtier du module de séparation 23 au moyen d'un connecteur. De même, la fibre de détection 7 peut être déconnectée et reconnectée non plus au boitier du module de balayage et d'injection, mais au boîtier du module de séparation 23 au moyen d'un connecteur. Le module de séparation 23 et le module de balayage et d'injection 2 sont conjugués optiquement par une fibre optique à deux cœurs 25.

La fibre à deux coeurs 25 comprend deux coeurs de fibres sensiblement concentriques, le premier coeur étant monomode et agencé pour transporter les faisceaux d'excitation 11, le deuxième coeur étant multimode et agencé pour transporter le flux lumineux collecté 14. Une des extrémités de la fibre à deux coeurs 25 peut être déconnectée et reconnectée du boîtier du module de séparation 23 au moyen d'un connecteur, et la deuxième extrémité de la fibre à deux coeurs 25 peut être déconnectée et reconnectée du boîtier du module de balayage et d'injection 2 au moyen d'un connecteur.

Comme dans le premier mode de réalisation, la fibre d'illumination 5 est agencée pour guider les faisceaux lumineux d'excitation 11. Le système optique 13 est agencé pour collimater les faisceaux d'excitation guidés par la fibre d'illumination 5. Le filtre dichroïque 20 est agencé pour diriger les faisceaux d'excitation 11 collimatés vers les moyens de balayage et d'injection 6, et pour diriger le flux collecté 14 vers le module de détection 4.

Contrairement au premier mode de réalisation, le filtre dichroïque 20 est agencé pour diriger les faisceaux d'excitation 11 vers le système optique 24 et la fibre à deux cœurs 25. Le système optique 24 est agencé pour focaliser les faisceaux d'excitation 11 en entrée de la fibre à deux cœurs 25 vers le module de balayage et d'injection 2. La fibre à deux cœurs 25 est agencée pour guider les faisceaux d'excitation 11 le long de son premier cœur jusqu'au module de balayage et d'injection 2. De plus, la fibre à deux cœurs est agencée pour guider le flux collecté 14 le long de son deuxième cœur jusqu'au module de séparation 23. Le système optique 24 est agencé pour collimater le flux collecté 14, et le filtre dichroïque 20 est agencé pour diriger le flux collecté 14 et collimaté vers la fibre de détection 7. Le système optique 19 est agencé pour focaliser le flux collecté 14 et réfléchi par le filtre 20 en entrée de la fibre de détection 7 vers le module de détection 4.

Le module de balayage et d'injection 2 comprend en outre un système optique 26 agencé pour collimater les faisceaux d'excitation 11 en provenance du module de séparation 23 et dirigés vers les moyens de balayage et d'injection 6, et pour focaliser le flux collecté 14 en entrée de la fibre à deux cœurs 25 vers le module de séparation 23.

Dans le dispositif 104, le module d'illumination 1 est conjugué optiquement au module de balayage et d'injection 2 par l'intermédiaire de la fibre optique d'illumination 5 et de la fibre à deux cœurs 25. De même, le module de détection 4 est conjugué optiquement au module de balayage et d'injection 2 par l'intermédiaire de la fibre optique de détection 7 et de la fibre à deux coeurs 25.

Le premier coeur de la fibre à deux coeurs 25 étant une fibre monomode gaussien, elle assure au faisceau d'excitation une grande qualité monomode gaussien, et un taux d'injection optimal des faisceaux d'excitation 11 dans le guide d'image 3. Ainsi, la fibre à deux coeurs à une fonction de filtrage modal des faisceaux d'excitation. De plus, le deuxième cœur étant multimode, la fibre à deux coeurs a pour fonction de ne pas effectuer de filtrage modal sur le flux collecté 14, pour les mêmes raisons que la fibre de détection 7.

Enfin, la fibre à deux cœurs 25 est agencée, pour une position donnée des moyens de balayage et d'injection 6, pour être conjuguée optiquement à une unique fibre du guide 3. En effet, pour une position donnée des moyens de balayage et d'injection 6, les faisceaux d'excitation 11 transportés par la fibre à deux cœurs 25 ne sont injectés que dans une unique fibre du guide, et le diamètre et la position du deuxième cœur sont calculés pour que le deuxième coeur transporte un flux lumineux collecté 14 n'ayant été guidé uniquement que le long de cette unique fibre du guide. Ainsi, la fibre à deux cœurs 25 a une fonction de filtrage spatial du flux collecté 14, et donne au dispositif 104 son caractère confocal.

Les caractéristiques techniques du filtre dichroïque 20 sont dépendantes des longueurs d'onde des faisceaux d'excitation 11 et du flux collecté 14. Un changement de longueur d'onde d'un des faisceaux d'excitation impose de remplacer le filtre multibande 20. Ce changement peut être réalisé facilement en remplaçant le module de séparation 23 par un nouveau module de séparation comprenant un nouveau filtre dichroïque 20, sans devoir réaligner optiquement le dispositif 104 ou un des modules du dispositif 104.

On va enfin décrire, en référence à la figure 6, un cinquième mode de réalisation de dispositif 105 selon l'invention. Le dispositif 105 ne sera décrit que pour ses différences par rapport au dispositif 104 décrit en référence à la figure 5. En particulier, les références 1 à 19, 21, 23 à 26, 108 à 110, 117 et 118 ne seront pas de nouveau décrites. Le dispositif 105 permet notamment la détection de signaux de fluorescence ou de réflectance collectés à l'extrémité distale du guide, et permet de construire des images de l'échantillon par fluorescence et par réflectance.

Dans le dispositif 105, le filtre dichroïque 20 du module de séparation 23 a été remplacé par le cube polarisant 21 décrit en référence à la figure 3. Comme pour le deuxième mode de réalisation de dispositif selon l'invention, le cube polarisant 21 permet d'utiliser un des détecteurs 18 et une des sources laser 8 pour de l'imagerie de réflectance. En effet, le cube polarisant 21 assure, comme décrit en référence aux figures 3 et 4, la réjection des réflexions parasites du faisceau d'excitation de la source laser 8 utilisée pour la réflectance. Pour ne pas perdre d'intensité des faisceaux d'excitation 11 dirigés vers les moyens de balayage et d'injection, toutes les fibres guidant les faisceaux d'excitation 11 jusqu'aux moyens de balayage et d'injection 6 (c'est-à-dire les fibres sources 108 à 110, la fibre d'illumination 5 et le premier coeur de la fibre à deux coeurs 25) sont des fibres à maintien de polarisation, ce qui entraîne un surcoût de réalisation.

Les dimensions typiques d'éléments des différents modes de réalisation venant d'être décrits sont :
- diamètre de cœur de la fibre de détection 7 : 50 micromètres,
- diamètre de cœur des fibres de démultiplexage 117, 118: 62,5 micromètres,
- diamètre de cœur de la fibre d'illumination 5 : 4 micromètres,
- diamètre du cœur monomode de la fibre à deux cœurs 25 : 4 micromètres, et
- diamètre du cœur multimode de la fibre à deux cœurs 25 : 10 micromètres

Bien sûr, l'invention n'est pas limitée aux exemples qui viennent d'être décrits et de nombreux aménagements peuvent être apportés à ces exemples sans sortir du cadre de l'invention tel que défini par les revendications.

En particulier, une source d'un faisceau d'excitation peut être un laser multi longueurs d'onde, c'est-à-dire un laser émettant simultanément plusieurs longueurs d'onde ou bandes de longueurs d'onde.

De même, une source d'un faisceau d'excitation peut-être un laser réglable en longueur d'onde, une Diode Electro Luminescente (DEL), une lampe à large spectre ou un super continuum.

De plus, les moyens de démultiplexage peuvent par exemple être du type phasar ou être fibrés, et comprendre par exemple une jonction fusionnant les cœurs des fibres de démultiplexage 117, 118 en le cœur de la fibre de détection 7. La description n'est nullement limitative concernant le nombre de sources par module d'illumination et le nombre de détecteurs par module de détection.

Le dispositif selon l'invention peut comprendre plusieurs multiplexeurs en série et/ou en parallèle entre les sources d'excitation et le module de balayage et d'injection.

Selon l'invention, les fibres du guide d'image peuvent être toutes multimodes.

Enfin, au moins un des filtres dichroïques d'un module de détection ou le filtre dichroïque d'un module de séparation peut être un filtre dichroïque dynamique dont on peut dynamiquement contrôler les bandes de longueur d'onde qu'il réfléchit ou qu'il transmet. Un tel filtre dichroïque dynamique peut par exemple comprendre un modulateur acousto-optique ou un modulateur électro-optique, une commande appliquée au modulateur permettant de contrôler les bandes de longueur d'onde.

## Revendications

1. Dispositif (101-105) d'imagerie, comprenant :
- un module d'illumination (1) comprenant des moyens pour émettre au moins un faisceau d'excitation (11),
- un module d'injection (2) comprenant un guide d'image (3) dont deux extrémités respectivement proximale (3a) et distale (3b) sont reliées par une pluralité de fibres optiques,
- un module de détection (4) comprenant des moyens (15-18, 117, 118) pour détecter un flux lumineux (14) collecté à l'extrémité distale (3b) du guide,
au moins un parmi le module d'illumination (1) et le module de détection (4) étant conjugué optiquement au module d'injection (2) par une fibre optique de conjugaison (5, 7, 25), le module d'injection étant un module de balayage et d'injection,
le module de balayage et d'injection comprenant en outre des moyens de balayage et d'injection (6), les moyens de balayage et d'injection (6) comprenant deux miroirs mobiles (6a) permettant un balayage bidimensionnel dans un plan de la surface d'entrée de l'extrémité proximale du guide (3), les moyens de balayage et d'injection étant agencés pour injecter l'au moins un faisceau d'excitation tour à tour dans une fibre du guide d'images (3) et du côté proximal (3a) du guide, la fibre optique de conjugaison (5, 7, 25) étant, pour une position donnée des moyens de balayage et d'injection (6), conjuguée optiquement à une unique fibre du guide (3),
le module de détection (4) étant conjugué optiquement au module de balayage et d'injection (2) par une fibre optique de détection (7),
les moyens de balayage et d'injection (6) étant agencés pour guider le flux lumineux collecté (14) à l'extrémité distale (3b) du guide (3) vers le module de détection (4),
la fibre optique de détection étant agencée pour être conjuguée optiquement à une unique fibre du guide,
la fibre optique de détection (7) étant agencée pour réaliser un filtrage spatial du flux lumineux collecté,
la fibre optique de détection (7) étant, pour une position donnée des moyens de balayage et d'injection (6), d'une part conjuguée optiquement à la fibre du guide dans laquelle les moyens de balayage et d'injection (6) sont agencés pour injecter tour à tour l'au moins un faisceau d'excitation (11), et d'autre part agencée pour rejeter la lumière provenant des autres fibres du guide (3),
le module d'illumination (1) étant conjugué optiquement au module de balayage et d'injection (2) par une fibre optique d'illumination (5), le dispositif comprenant des moyens de séparation (20, 21) agencés pour diriger l'au moins un faisceau d'excitation (11) vers les moyens de balayage et d'injection (6), et pour diriger vers le module de détection (4) le flux lumineux collecté (14) provenant des moyens de balayage et d'injection (6),
**caractérisé en ce que**
le guide d'image (3) a ses deux extrémités respectivement proximale (3a) et distale (3b) reliées par une pluralité de fibres optiques multimodes,
la fibre optique de détection (7) est une fibre optique multimode,
la fibre optique d'illumination (5) est une fibre optique monomode,
les moyens de séparation (20, 21) font partie d'un module de séparation (23), le module de détection (4) et le module d'illumination (1) étant conjugués optiquement au module de balayage et d'injection (2) par le module de séparation (23) et une fibre optique de séparation (25) conjuguant le module de séparation (23) au module de balayage et d'injection (2),
la fibre optique de séparation (25) comprenant deux cœurs de fibres sensiblement concentriques, le premier cœur étant monomode et agencé pour transporter l'au moins un faisceau d'excitation (11), le deuxième cœur étant multimode et agencé pour transporter le flux lumineux collecté (14).

2. Dispositif selon la revendication 1, **caractérisé en ce que** le guide d'image (3) est déconnectable d'un boîtier du module de balayage et d'injection (2).

3. Dispositif selon la revendication 1 ou 2, **caractérisé en ce qu'**il comprend des moyens pour déconnecter et reconnecter la conjugaison par la fibre optique de conjugaison (5, 7, 25).

4. Dispositif selon l'une quelconque des revendications précédentes, **caractérisé en ce que** la fibre optique d'illumination est agencée pour être conjuguée optiquement tour à tour à une unique fibre du guide dans laquelle est injecté tour à tour l'au moins un faisceau d'excitation par les moyens de balayage et d'injection.

5. Dispositif selon l'une quelconque des revendications précédentes, **caractérisé en ce qu'**il comprend des moyens pour déconnecter et reconnecter la conjugaison par la fibre optique d'illumination (5).

6. Dispositif selon l'une quelconque des revendications précédentes , **caractérisé en ce que** les moyens d'émission comprennent plusieurs sources (8-10) émettant chacune un faisceau d'excitation, et **en ce que** le module d'illumination comprend des moyens pour multiplexer (12) les faisceaux d'excitation (11) dans la fibre optique d'illumination (5).

7. Dispositif selon la revendication 6, **caractérisé en ce que** chaque source (8-10) est conjuguée optiquement aux moyens de multiplexage (12) par une fibre optique source (108-110), et **en ce que** les moyens de multiplexage comprennent un multiplexeur fibré (12) fusionnant les cœurs des fibres sources (108-110).

8. Dispositif selon l'une quelconque des revendications précédentes, **caractérisé en ce qu'**il comprend des moyens pour déconnecter et reconnecter la conjugaison par la fibre optique de détection (7).

9. Dispositif selon l'une quelconque des revendications précédentes, **caractérisé en ce que** le module de détection (4) comprend des moyens (15) pour démultiplexer en longueur d'onde le flux lumineux collecté (14).

10. Dispositif selon la revendication 9, **caractérisé en ce que** les moyens de détection (4) comprennent plusieurs détecteurs (17, 18), chaque détecteur étant agencé pour détecter une bande de longueur d'onde donnée du flux démultiplexé (14).

11. Dispositif selon l'une quelconque des revendications 1 à 10, **caractérisé en ce que** les moyens de séparation comprennent un filtre dichroïque (20), de préférence multibande.

12. Dispositif selon l'une quelconque des revendications 1 à 10, **caractérisé en ce que** les moyens de séparation comprennent un cube séparateur (21), le module d'illumination (1) et le module de détection étant de préférence prévus pour de l'imagerie par réflectance.

13. Procédé d'imagerie, comprenant :
- une émission, par un module d'illumination (1), d'au moins un faisceau d'excitation (11),
- une injection, par un module d'injection (2), de l'au moins un faisceau d'excitation (11) dans un guide d'image (3) comprenant deux extrémités respectivement proximale (3a) et distale (3b) reliées par une pluralité de fibres optiques et du côté proximal (3a) du guide,
- une détection, par un module de détection (4), d'un flux lumineux (14) collecté à l'extrémité distale (3b) du guide,
- une conjugaison optique, par une fibre optique de conjugaison (5, 7, 25), du module d'illumination (1) et/ou du module de détection (4) avec le module de balayage et d'injection (2),
le module d'injection (2) étant un module de balayage et d'injection de sorte que l'injection est une injection de l'au moins un faisceau d'excitation (11) tour à tour dans une fibre du guide d'image (3) du côté proximal (3a) du guide,
la conjugaison optique avec le module de balayage et d'injection (2) comprenant une conjugaison optique de la fibre optique de conjugaison (5, 7, 25) avec une unique fibre du guide (3),
ledit procédé comprenant un balayage bidimensionnel dans un plan de la surface d'entrée de l'extrémité proximale du guide par deux miroirs mobiles (6a) des moyens de balayage et d'injection,
la conjugaison optique avec le module de balayage et d'injection comprenant un guidage du flux collecté (14), du module de balayage et d'injection (2) vers le module de détection (4) le long d'une fibre optique de détection (7),
les moyens de balayage et d'injection (6) guidant le flux lumineux collecté (14) à l'extrémité (3b) du guide (3) vers le module de détection (4),
la conjugaison optique avec le module de balayage et d'injection (2) comprenant une conjugaison optique de la fibre optique de détection (7) avec une unique fibre du guide (3),
le procédé comprenant un filtrage spatial, par la fibre de détection, du flux collecté (14), de sorte que la fibre optique de détection (7) soit, pour une position donnée des moyens de balayage et d'injection (6), d'une part conjuguée optiquement à la fibre du guide dans laquelle les moyens de balayage et d'injection (6) injectent tour à tour l'au moins un faisceau d'excitation (11), et d'autre part rejette la lumière provenant des autres fibres du guide (3),
la conjugaison optique avec le module de balayage et d'injection comprenant un guidage de l'au moins un faisceau d'excitation (11), du module d'illumination (1) vers le module de balayage et d'injection (2) le long d'une fibre optique d'illumination (5),
des moyens de séparation (20, 21) dirigeant l'au moins un faisceau d'excitation (11) vers les moyens de balayage et d'injection (6) et dirigeant vers le module de détection (4) le flux lumineux collecté (14) provenant des moyens de balayage et d'injection (6),
**caractérisé en ce que**
le guide d'image (3) a ses deux extrémités respectivement proximale (3a) et distale (3b) reliées par une pluralité de fibres optiques multimodes,
la fibre optique de détection (7) est une fibre optique multimode,
la fibre optique d'illumination (5) est une fibre optique monomode,
les moyens de séparation (20, 21) font partie d'un module de séparation (23), le module de détection (4) et le module d'illumination (1) étant conjugués optiquement au module de balayage et d'injection (2) par le module de séparation (23) et une fibre optique de séparation (25) conjuguant le module de séparation (23) au module de balayage et d'injection (2),
la fibre optique de séparation (25) comprenant deux cœurs de fibres sensiblement concentriques, le premier cœur étant monomode et transportant l'au moins un faisceau d'excitation (11), le deuxième cœur étant multimode et transportant le flux lumineux collecté (14).

14. Procédé selon la revendication 13, **caractérisé en ce que** le guide d'image (3) est déconnectable d'un boîtier du module de balayage et d'injection (2).

15. Procédé selon la revendication 13 ou 14, **caractérisé en ce que** la conjugaison optique avec le module de balayage et d'injection (2) comprend une conjugaison optique de la fibre optique d'illumination (5) avec une unique fibre du guide (3).

16. Procédé selon l'une quelconque des revendications 13 à 15, **caractérisé en ce qu'**il comprend une superposition spatiale de plusieurs faisceaux d'excitation (11) dans la fibre optique d'illumination (5).

## Patentansprüche

1. Vorrichtung (101-105) zur Bildgebung, umfassend:
- ein Beleuchtungsmodul (1) umfassend Mittel zum Aussenden mindestens eines Anregungsstrahls (11),
- ein Einleitungsmodul (2) umfassend einen Bildleiter (3), dessen zwei proximale (3a) bzw. distale (3b) Enden durch eine Vielzahl von optischen Fasern verbunden sind,
- ein Detektionsmodul (4) umfassend Mittel (15-18, 117, 118) zum Detektieren eines am distalen Ende (3b) des Leiters gesammelten Lichtstroms (14),
wobei mindestens eines von dem Beleuchtungsmodul (1) und dem Detektionsmodul (4) mit dem Einleitungsmodul (2) durch eine optische Verbindungsfaser (5, 7, 25) optisch verbunden ist,
wobei das Einleitungsmodul ein Abtast- und Einleitungsmodul ist, wobei das Abtast- und Einleitungsmodul ferner Abtast- und Einleitungsmittel (6) umfasst, wobei die Abtast- und Einleitungsmittel (6) zwei bewegbare Spiegel (6a) umfassen, die eine zweidimensionale Abtastung in einer Ebene der Eintrittsfläche des proximalen Endes des Leiters (3) ermöglichen, wobei die Abtast- und Einleitungsmittel dazu eingerichtet sind, den mindestens einen Anregungsstrahl der Reihe nach in eine Faser des Bildleiters (3) auf der proximalen Seite (3a) des Leiters einzuleiten,
wobei die optische Verbindungsfaser (5, 7, 25) für eine gegebene Position der Abtast- und Einleitungsmittel (6) optisch mit einer einzelnen Faser des Leiters (3) verbunden ist,
wobei das Detektionsmodul (4) durch eine optische Detektionsfaser (7) optisch mit dem Abtast- und Einleitungsmodul (2) verbunden ist,
wobei die Abtast- und Einleitungsmittel (6) dazu eingerichtet sind, den am distalen Ende (3b) des Leiters (3) gesammelten Lichtstrom (14) zum Detektionsmodul (4) zu leiten,
wobei die optische Detektionsfaser dazu eingerichtet ist, optisch mit einer einzelnen Faser des Leiters verbunden zu werden,
wobei die optische Detektionsfaser (7) dazu eingerichtet ist, eine räumliche Filterung des gesammelten Lichtstroms zu realisieren,
wobei die optische Detektionsfaser (7) für eine gegebene Position der Abtast- und Einleitungsmittel (6) einerseits optisch mit der Faser des Leiters verbunden ist, bei der die Abtast- und Einleitungsmittel (6) dazu eingerichtet sind, der Reihe nach den mindestens einen Anregungsstrahl (11) einzuleiten, und andererseits dazu eingerichtet ist, das von anderen Fasern des Leiters (3) stammende Licht abzuweisen,
wobei das Beleuchtungsmodul (1) mit dem Abtast- und Einleitungsmodul (2) durch eine optische Beleuchtungsfaser (5) optisch verbunden ist,
wobei die Vorrichtung Trennmittel (20, 21) umfasst, die dazu eingerichtet sind, den mindestens einen Anregungsstrahl (11) zu den Abtast- und Einleitungsmitteln (6) zu lenken und den gesammelten Lichtstrom (14), der von den Abtast- und Einleitungsmitteln (6) stammt, zum Detektionsmodul (4) zu lenken,
**dadurch gekennzeichnet, dass**
der Bildleiter (3) seine zwei proximalen (3a) bzw. distalen (3b) Enden durch eine Vielzahl von multimodalen optischen Fasern verbunden aufweist,
die optische Detektionsfaser (7) eine multimodale optische Faser ist,
die optische Beleuchtungsfaser (5) eine monomodale optische Faser ist,
die Trennmittel (20, 21) einen Teil eines Trennmoduls (23) darstellen, wobei das Detektionsmodul (4) und das Beleuchtungsmodul (1) durch das Trennmodul (23) und eine optische Trennfaser (25), die das Trennmodul (23) mit dem Abtast- und Einleitungsmodul (2) verbindet, optisch mit dem Abtast- und Einleitungsmodul (2) verbunden sind,
wobei die optische Trennfaser (25) zwei im Wesentlichen konzentrische Faserkerne aufweist, wobei der erste Kern monomodal und dazu eingerichtet ist, den mindestens einen Anregungsstrahl (11) zu übertragen, und der zweite Kern multimodal und dazu eingerichtet ist, den gesammelten Lichtstrom (14) zu übertragen.

2. Vorrichtung nach Anspruch 1, **dadurch gekennzeichnet, dass** der Bildleiter (3) von einem Gehäuse des Abtast- und Einleitungsmoduls (2) abtrennbar ist.

3. Vorrichtung nach Anspruch 1 oder 2, **dadurch gekennzeichnet, dass** diese Mittel zum Unterbrechen und Wiederverbinden der Verbindung durch die optische Verbindungsfaser (5, 7, 25) umfasst.

4. Vorrichtung nach einem der vorhergehenden Ansprüche, **dadurch gekennzeichnet, dass** die optische Beleuchtungsfaser dazu eingerichtet ist, der Reihe nach mit einer einzelnen Faser des Leiters optisch verbunden zu werden, in die der mindestens eine Anregungsstrahl durch die Abtast- und Einleitungsmittel der Reihe nach eingeleitet wird.

5. Vorrichtung nach einem der vorhergehenden Ansprüche, **dadurch gekennzeichnet, dass** diese Mittel zum Unterbrechen und Wiederverbinden der Verbindung durch die optische Beleuchtungsfaser (5) umfasst.

6. Vorrichtung nach einem der vorhergehenden Ansprüche, **dadurch gekennzeichnet, dass** die Aussendemittel mehrere Quellen (8-10) umfassen, die jeweils einen Anregungsstrahl aussenden, und dass das Beleuchtungsmodul Mittel zum Multiplexen (12) der Anregungsstrahlen (11) in die optische Beleuchtungsfaser (5) umfasst.

7. Vorrichtung nach Anspruch 6, **dadurch gekennzeichnet, dass** jede Quelle (8-10) durch eine optische Quellenfaser (108-110) optisch mit den Multiplexmitteln (12) verbunden ist, und dass die Multiplexmittel einen Fasermultiplexer (12) umfassen, der die Kerne der Quellenfasern (108-110) zusammenführt.

8. Vorrichtung nach einem der vorhergehenden Ansprüche, **dadurch gekennzeichnet, dass** diese Mittel zum Unterbrechen und Wiederverbinden der Verbindung durch die optische Detektionsfaser (7) umfasst.

9. Vorrichtung nach einem der vorhergehenden Ansprüche, **dadurch gekennzeichnet, dass** das Detektionsmodul (4) Mittel (15) zum Demultiplexen des gesammelten Lichtstroms (14) nach Wellenlänge umfasst.

10. Vorrichtung nach Anspruch 9, **dadurch gekennzeichnet, dass** die Detektionsmittel (4) mehrere Detektoren (17, 18) umfassen, wobei jeder Detektor dazu eingerichtet ist, ein bestimmtes Wellenlängenband des demultiplexierten Lichtstroms (14) zu detektieren.

11. Vorrichtung nach einem der Ansprüche 1 bis 10, **dadurch gekennzeichnet, dass** die Trennmittel ein dichroitisches Filter (20), vorzugsweise ein Mehrbandfilter, umfassen.

12. Vorrichtung nach einem der Ansprüche 1 bis 10, **dadurch gekennzeichnet, dass** die Trennmittel einen Trennwürfel (21) umfassen, wobei das Beleuchtungsmodul (1) und das Detektionsmodul vorzugsweise zur Reflexionsbildgebung vorgesehen sind.

13. Verfahren zur Bildgebung, umfassend:
- eine Aussendung mindestens eines Anregungsstrahls (11) durch ein Beleuchtungsmodul (1),
- eine Einleitung des mindestens einen Anregungsstrahls (11) durch ein Einleitungsmodul (2) in einen Bildleiter (3), der zwei proximale (3a) bzw. distale (3b) Enden aufweist, die durch eine Vielzahl von optischen Fasern verbunden sind, auf der proximalen Seite (3a) des Leiters,
- eine Detektion eines am distalen Ende (3b) des Leiters gesammelten Lichtstroms (14) durch ein Detektionsmodul (4),
- eine optische Verbindung des Beleuchtungsmoduls (1) und/oder des Detektionsmoduls (4) mit dem Abtast- und Einleitungsmodul (2) durch eine optische Verbindungsfaser (5, 7, 25),
wobei das Einleitungsmodul (2) ein Abtast- und Einleitungsmodul ist, derart, dass die Einleitung eine Einleitung des mindestens einen Anregungsstrahls (11) der Reihe nach in eine Faser des Bildleiters (3) auf der proximalen Seite (3a) des Leiters ist,
wobei die optische Verbindung mit dem Abtast- und Einleitungsmodul (2) eine optische Verbindung der optischen Verbindungsfaser (5, 7, 25) mit einer einzelnen Faser des Leiters (3) umfasst,
wobei das Verfahren ein zweidimensionales Abtasten in einer Ebene der Eintrittsfläche des proximalen Endes des Leiters durch zwei bewegbare Spiegel (6a) der Abtast- und Einleitungsmittel umfasst,
wobei die optische Verbindung mit dem Abtast- und Einleitungsmodul eine Leitung des gesammelten Flusses (14) von dem Abtast- und Einleitungsmodul (2) zu dem Detektionsmodul (4) entlang einer optischen Detektionsfaser (7) umfasst,
wobei die Abtast- und Einleitungsmittel (6) den gesammelten Lichtstrom (14) am Ende (3b) des Leiters (3) zum Detektionsmodul (4) leiten,
wobei die optische Verbindung mit dem Abtast- und Einleitungsmodul (2) eine optische Verbindung der optischen Detektionsfaser (7) mit einer einzelnen Faser des Leiters (3) umfasst,
wobei das Verfahren eine räumliche Filterung des gesammelten Flusses (14) durch die Detektionsfaser umfasst, derart, dass die optische Detektionsfaser (7) für eine gegebene Position der Abtast- und Einleitungsmittel (6) einerseits optisch mit der Faser des Leiters verbunden ist, bei der die Abtast- und Einleitungsmittel (6) der Reihe nach den mindestens einen Anregungsstrahl (11) einleiten, und andererseits das von anderen Fasern des Leiters (3) stammende Licht abweist,
wobei die optische Verbindung mit dem Abtast- und Einleitungsmodul eine Leitung des mindestens einen Anregungsstrahls (11) von dem Beleuchtungsmodul (1) zu dem Abtast- und Einleitungsmodul (2) entlang einer optischen Beleuchtungsfaser (5) umfasst,
wobei Trennmittel (20, 21) den mindestens einen Anregungsstrahl (11) zu den Abtast- und Einleitungsmitteln (6) lenken und den von den Abtast- und Einleitungsmitteln (6) stammenden gesammelten Lichtstrom (14) zum Detektionsmodul (4) lenken,
**dadurch gekennzeichnet, dass**
der Bildleiter (3) seine zwei proximalen (3a) bzw. distalen (3b) Enden durch eine Vielzahl von multimodalen optischen Fasern verbunden aufweist,
die optische Detektionsfaser (7) eine multimodale optische Faser ist,
die optische Beleuchtungsfaser (5) eine monomodale optische Faser ist,
die Trennmittel (20, 21) einen Teil eines Trennmoduls (23) darstellen, wobei das Detektionsmodul (4) und das Beleuchtungsmodul (1) durch das Trennmodul (23) und eine optische Trennfaser (25), die das Trennmodul (23) mit dem Abtast- und Einleitungsmodul (2) verbindet, optisch mit dem Abtast- und Einleitungsmodul (2) verbunden sind,
wobei die optische Trennfaser (25) zwei im Wesentlichen konzentrische Faserkerne aufweist, wobei der erste Kern monomodal ist und den mindestens einen Anregungsstrahl (11) überträgt, und der zweite Kern multimodal ist und den gesammelten Lichtstrom (14) überträgt.

14. Verfahren nach Anspruch 13, **dadurch gekennzeichnet, dass** der Bildleiter (3) von einem Gehäuse des Abtast- und Einleitungsmoduls (2) abtrennbar ist.

15. Verfahren nach Anspruch 13 oder 14, **dadurch gekennzeichnet, dass** die optische Verbindung mit dem Abtast- und Einleitungsmodul (2) eine optische Verbindung der optischen Beleuchtungsfaser (5) mit einer einzelnen Faser des Leiters (3) umfasst.

16. Verfahren nach einem der Ansprüche 13 bis 15, **dadurch gekennzeichnet, dass** dieses eine räumliche Überlagerung von mehreren Anregungsstrahlen (11) in der optischen Beleuchtungsfaser (5) umfasst.

## Claims

1. Imaging device (101-105) comprising:
- an illumination module (1) comprising means for emitting at least one excitation beam (11),
- an injection module (2) comprising an image guide (3) whose two respective proximal (3a) and distal (3b) ends are linked by a plurality of optical fibers,
- a detection module (4) comprising means (15-18, 117, 118) for detecting a luminous flux (14) collected at the distal end (3b) of the guide,
at least either the illumination module (1) or the detection module (4) being optically conjugated with the injection module (2) by a conjugating optical fiber (5, 7, 25) the injection module being a scanning and injection module,
the scanning and injection module further comprising scanning and injection means (6), the scanning and injection means (6) comprising two moving mirrors (6a) which allow two-dimensional scanning in a plane of the input surface of the proximal end of the guide (3), the scanning and injection means being arranged for alternately injecting the at least one excitation beam into a fiber of the image guide (3) from the proximal end (3a) of the guide,
the optical conjugation fiber (5, 7, 25) being, for a given position of the scanning and injection means (6), optically conjugated with a single fiber of the guide (3),
the detection module (4) being optically conjugated with the scanning and injection module (2) by a detecting optical fiber (7),
the scanning and injection means (6) being arranged for guiding the luminous flux (14) collected from the distal end (3b) of the guide (3) to the detection module (4), the detecting optical fiber being arranged for being optically conjugated with a single fiber of the guide,
the detecting optical fiber (7) being made to perform a spatial filtering of the collected luminous flux,
the detecting optical fiber (7) being, for a given position of the scanning and injection means (6):
- optically conjugated with the fiber of the guide into which the scanning and injection means (6) are made to inject the at least one excitation beam (11), and
- arranged to reject the light coming from the other fibers of the guide (3),
the illumination module (1) being optically conjugated with the scanning and injection module (2) by an illuminating optical fiber (5),
the device comprising splitting means (20, 21) made to direct the at least one excitation beam (11) to the scanning and injection means (6) and to direct to the detection module (4) the collected luminous flux (14) coming from the scanning and injection means (6)
**characterized in that**
the image guide (3) has its two respective proximal (3a) and distal (3b) ends linked by a plurality of multimode optical fibers,
the detecting optical fiber (7) being a multi-mode optical fiber,
the illuminating optical fiber (5) being a single-mode optical fiber,
the splitting means (20, 21) being part of a splitting module (23), the detection module (4) and the illumination module (1) being optically conjugated with the scanning and injection module (2) by the splitting module (23) and a splitting optical fiber (25) that conjugates the splitting module (23) with the scanning and injection module (2),
the splitting optical fiber (25) comprises two substantially concentric fiber cores, the first core being single-mode and made to transport the at least one excitation beam (11), the second core being multi-mode and made to transport the collected luminous flux (14).

2. Device according to claim 1, **characterized in that** the image guide (3) is disconnectable from a housing of the scanning and injection module (2).

3. Device according to claim 1 or 2, **characterized in that** it comprises means for disconnecting and reconnecting the conjugation via the conjugating optical fiber (5,7,25).

4. Device according to any of the previous claims, **characterized in that** the illuminating optical fiber is made to be alternately optically conjugated with a single fiber of the guide into which the at least one excitation beam is alternatly injected by the scanning and injection means.

5. Device according to any of the previous claims, **characterized in that** it comprises means for disconnecting and reconnecting the conjugation via the illuminating optical fiber (5).

6. Device according to any of the previous claims, **characterized in that** the emitting means comprise several sources (8-10), each emitting an excitation beam, and **in that** the illumination module comprises means for multiplexing (12) the excitation beams (11) in the illuminating optical fiber (5).

7. Device according to claim 6, **characterized in that** each source (8-10) is optically conjugated with the multiplexing means (12) by a source optical fiber (108-110), and **in that** the multiplexing means comprise a fiber multiplexer (12) that fuses the cores of the source fibers (108-110).

8. Device according to any of the previous claims, **characterized in that** it comprises means for disconnecting and reconnecting the conjugation via the detection optical fiber (7).

9. Device according to any of the previous claims, **characterized in that** the detection module (4) comprises means (15) for wavelength demultiplexing the collected luminous flux (14).

10. Device according to claim 9, **characterized in that** the detection means (4) comprise several detectors (17, 18), each detector being made to detect a given spectral bandwidth of the demultiplexed flux (14).

11. Device according to any of the previous claims 1 to 10, **characterized in that** the splitting means comprise a dichroic filter (20), preferably a multiband filter.

12. Device according to any of the previous claims 1 to 10, **characterized in that** the splitting means comprise a beam-splitting cube (21), the illumination module (1) and the detection module preferably being provided for reflectance imaging.

13. Imaging method, comprising:
- an emission, by an illumination module (1), of at least one excitation beam (11),
- an injection, by an injection module (2), of the at least one excitation beam (11) into an image guide (3) comprising two respective proximal (3a) and distal (3b) ends linked by a plurality of optical fibers, from the proximal end (3a) of the guide,
- a detection, by a detection module (4), of a luminous flux (14) collected at the distal end (3b) of the guide, and
- an optical conjugation, by a conjugating optical fiber (5, 7, 25), of the illumination module (1) and/or the detection module (4) with the scanning and injection module (2),
the injection module (2) being a scanning and injection module in such a way that the injection is an injection of the at least one excitation beam (11) alternately into one fiber of the image guide (3) from the proximal end (3a) of the guide,
the optical conjugation with the fiber injection module (2) comprising an optical conjugation of the conjugating optical fiber (5, 7, 25) with a single fiber of the guide (3),
the method comprising a two-dimensional scanning in a plane of the input surface of the proximal end of the guide (3) by two moving mirrors (6a) of the scanning and injection means,
the optical conjugation with the scanning and injection module comprising guiding the collected luminous flux (14) from the scanning and injection module (2) to the detection module (4) along a detection optical fiber (7),
the scanning and injection means (6) guiding the luminous flux (14) collected from the end (3b) of the guide (3) to the detection module (4),
the optical conjugation with the scanning and injection module (2) comprising an optical conjugation of the detecting optical fiber (7) with a single fiber of the guide (3),
the method comprising a spatial filtering, by the detecting optical fiber, of the collected luminous flux (14), in such a way that the detecting optical fiber (7) is, for a given position of the scanning and injection means (6):
- optically conjugated with the fiber of the guide into which the scanning and injection means (6) alternately inject the at least one excitation beam (11), and
- rejecting the light coming from the other fibers of the guide (3),
the optical conjugation with the scanning and injection module comprising a guiding of the at least one excitation beam (11), from the illumination module (1) to the scanning and injection module (2) along an illuminating optical fiber (5),
splitting means (20, 21) directing the at least one excitation beam (11) to the scanning and injection means (6) and directing the collected luminous flux (14) coming from the scanning and injection means (6) to the detection module (4),
**characterized in that**
the image guide (3) has its two respective proximal (3a) and distal (3b) ends linked by a plurality of multimode optical fibers,
the detecting optical fiber (7) being a multi-mode optical fiber,
the illuminating optical fiber (5) being a single-mode optical fiber,
the splitting means (20, 21) being part of a splitting module (23), the detection module (4) and the illumination module (1) being optically conjugated with the scanning and injection module (2) by the splitting module (23) and a splitting optical fiber (25) that conjugates the splitting module (23) with the scanning and injection module (2),
the splitting optical fiber (25) comprising two substantially concentric fiber cores, the first core being single-mode and transporting the at least one excitation beam (11), the second core being multi-mode and transporting the collected luminous flux (14).

14. Method according to claim 13, **characterized in that** the image guide (3) is disconnectable from a housing of the scanning and injection module (2).

15. Method according to claim 13 or 14, **characterized in that** the optical conjugation with the scanning and injection module (2) comprises an optical conjugation of the illuminating optical fiber (5) with a single fiber of the guide (3).

16. Method according to any one of claims 13 to 15, **characterized in that** it comprises a spatial superposition of several excitation beams (11) in the illuminating optical fiber (5).
